# EUROPEAN PATENT SPECIFICATION

(11) **EP 4 008 298 B1**
(45) Date of publication and mention of the grant of the patent: **07.02.2024**
(21) Application number: 20383054.2
(22) Date of filing: 03.12.2020
(51) Int. Cl.: A61F 13/00

(54) **TATTOO PROTECTOR BANDAGE AND USE AS A TATTOO PROTECTOR**
TATTOO-SCHUTZ-BANDAGE UND VERWENDUNG ALS TATTOO-SCHUTZ
BANDAGE PROTECTEUR DE TATOUAGE ET UTILISATION COMME PROTECTEUR DE TATOUAGE

(43) Date of publication of application: 08.06.2022
(73) Proprietor: Lidermed, S.A., 08302 Mataró (ES)
(72) Inventor: DOMÈNECH LLOBET, Xavier, 08302 MATARÓ (ES)
(74) Representative: Manresa Medina, José Manuel

(56) References cited:
- EP-A1- 3 420 830
- US-B2- 7 937 973

## Description

Tattoo protector bandage and use as a tattoo protector.

Tattoo protector bandage and use as a tattoo protector, of the type having a tubular configuration, with at least one opening for introducing a member of the human body, forming an inner side which is in contact with the skin and an outer side; characterised in that, in its total composition it comprises 90-94% viscose, 4-6% elastane and 2-4% polyamide.

### BACKGROUND OF THE INVENTION

Covering or protective elements for skin tattoos are known in the state of the art.

State of the art is Spain Utility Patent n° 9602349 (ES1035074) "FLEXIBLE, BREATHABLE AND WATERPROOF PLASTIC ADHESIVE PATCH FOR SOLAR TATTOO ON THE SKIN BY COVERING THE SKIN (*PARCHE PLASTICO, ADHESIVO, FLEXIBLE, TRANSPIRABLE E IMPERMEABLE PARA TATUAJE SOLAR EN LA PIEL POR CUBRICION DE LA PIEL*"), in the name of José Maria EXPOSITO PRECIADO, of the year 1996, which refers to a plastic adhesive patch for skin solar tattoo made of plastic material, being flexible, breathable, waterproof and having an adhesive suitable for human use. Placed on the skin, the patch protects against the sun rays, UVA rays or colorant products. The so-called solar tattoo is accomplished after a certain period of time, when the skin has avoided pigmentation for having been covered by the patch; the tattoo then stands out from the rest of the pigmented skin.

Also known is European Patent N° 1183157 (ES2207345) "DEVICE FOR APPLYING AN ADHESIVE (TEMPORARY) SKIN TATTOO AND FOR ADMINISTERING A PHARMACEUTICAL AND/OR COSMETIC PRODUCT in the name MARTEL, STEPHANE CHRISTOPHER, of the year 1999, which refers to a device for applying an adhesive temporary tattoo comprising a soft support that has two main opposite faces, one of the faces having an opening covered by an applicator pad having a temporary skin adhesive tattoo, while the other face carries a deformable liquid reservoir. The invention is characterised in that a sealed intermediate cavity is formed above the applicator pad; the liquid reservoir, arranged outside said opening is substantially rectangular, oval or oblong in shape; and the liquid reservoir and the intermediate cavity are designed to communicate with each other through a passage; said passage extends along a width that corresponds with the larger side of the reservoir and also comprises a reduction area which, in turn, extends along a width corresponding to the largest side of the reservoir. The invention also comprises a sealing means. Said sealing means is designed to be torn out or detached in order to release the fluid from the reservoir in a direction towards the applicator pad through the intermediate cavity when sufficient pressure is exerted on the liquid reservoir in order to moisten the applicator pad provided on the outer surface of a temporary skin adhesive tattoo.

Worth mentioning is European Patent 2549966 "COMPRESSION TEXTILE COMPRISING CHITOSAN COATED FABRICS", of the year 2011, in the name SIGVARIS AG, concerning a compression textile comprising at least one fibre containing chitosan, wherein the chitosan-containing fibre is a cellulose-based fibre, e.g., a viscose, modal, polynosic or lyocell fibre whose surface is at least partially coated with chitosan; the chitosan-coated fibre covers an elastic yarn.

Known is also that tattooists commonly apply a gauze over the tattoo and, subsequently, a plastic film to wrap the tattooed member or the tattooed part.

Last, it is worth pointing out that Patent US2013323452 "Self-adhering cover for temporarily and incrementally concealing a tattoo" in the name SANDMARK, RICHARD SCOTT et. al., of the year 2012, which refers to a self-adhering cover for temporarily concealing a tattoo while matching the tone of the skin of a user. The self-adhering cover includes a plurality of stacked layers. The plurality of stacked layers is for adhering to the user's skin on the tattoo and, in so doing, temporarily and incrementally concealing said tattoo while at the same time matching the user's skin tone so as to allow the self-adhering cover to be relatively invisible.

### BRIEF DESCRIPTION OF THE INVENTION

The present invention falls within the field of devices for covering artistic works of the skin, such as, for example, tattoos.

Specifically, the invention is directed to a sector that does not need medical stuff nor medical knowledge to implement the tattoo; rather, the professional is an artist, a person who creates works of art on the human body, to embellish and improve the aesthetic look of a person.

The nearest document is the use of film and gauze for protecting the tattoos. The person skilled in this art is the professional tattooist, who makes the tattoo and uses protection for the tattoo.

Said use solves the problem of protecting the tattoo against harshness in an undetachable manner.

However, this practice has the disadvantage that the film causes the occlusion of the skin, which in turn causes the skin to expel the ink and the tattoo to deteriorate due to the tattoo being very fragile in its first 24 hours.

In addition, there is a risk that if there is a small injury, the injury will not heal, as it is prevented from breathing.

Last, upon applying the film on the member, the pressure exerted on the skin can cause that the skin stays compressed, which will ruin the tattoo.

This invention solves the above-stated problems by using a tubular bandage having a combination of viscose, elastane and polyamide that prevents applying too much pressure on the tattooed member or on the tattooed part of the body, and therefore it prevents the skin from expelling more ink than necessary.

In effect, the tattoo expels a small quantity of ink, which the tattooist already expects. This invention prevents that the skin expels more ink than the strictly necessary quantity expected by the tattooist.

In addition, the bandage protects the tattoo from friction, dirt and sunlight without asphyxiating the skin. Unlike the nearest reference, the skin in this invention breathes and is not soaked, since soaking makes the skin expel more ink than desired, thus damaging the tattoo.

Thus, this invention allows the tattoo to dry and to attach the work as created by the tattooist artist to the skin without the skin expelling, when being compressed, the tattoo ink, with the resulting damage to the tattoo.

In addition, being breathable and therefore allowing moist control, the bandage has also anti-bacterial properties.

The object of the present invention is a tattoo protective bandage, of the type having a tubular configuration, and at least one opening for introducing a member of the human body, forming an inner side, which is in contact with the skin, and an outer side; characterised in that it comprises, out over the total, 90-94% of viscose, 4-6% of elastane and 2-4% polyamide.

An additional object of the present invention is a bandage according to claim 1 or 2 for use as tattoo protector.

### SPECIFIC EMBODIMENT OF THE PRESENT INVENTION

Thus, the tattoo protecting bandage has a tubular configuration and at least one opening for introducing a member of the human body. It could have an inlet and an outlet, for example, for inserting the arm, or just one inlet, and be configured as a sock.

The bandage has an inner face, which is in contact with the skin, and an outer face, which is visible from the outsider.

It comprises:
- over the total, 90-94% of viscose
- over the total 4-6% of elastane
- over the total 2-4% of polyamide

The above percentages refer comparatively to the total fibres.

Viscose is obtained from natural cellulose, which is found in wood pulp, which makes viscose an eco-friendly and bio-degradable material.

Elastane provides a small amount of elasticity, and polyamide tends to shrink progressively, which favours recovering the bandage elasticity thanks to the polyamide, bit by bit, without excessively pressing the user's skin. Moreover, polyamide coats the elastane.

The combination of viscose, elastane and polyamide in these proportions and, if the bandage size is adequately chosen, prevents exerting excessive pressure and therefore prevents that the ink applied on the skin be not expelled if too much pressure is exerted on the skin.

This bandage perspires naturally, and it has been estimated that it absorbs approximately 50% more moisture than cotton thanks to the proportion of viscose content and to its natural nature, all of which endows it with anti-bacterial properties.

The final product is a soft, breathable, weightless and comfortable bandage, with an extremely smooth surface and great adaptability to the part of the body to be covered.

Optionally, the bandage may comprise a dermatological product in its inner face, to help heal the skin in case the tattoo has made the skin too sensitive.

In addition, the bandage helps to absorb the dermatological product, for instance a cream, into the skin.

Bandage for use as a tattoo protector.

This bandage helps to protect the skin during the first 24 hours by preventing the skin from rubbing against clothing (the skin may be irritated due to the tattoo) and also protects the skin from a harsh weather.

In effect, the bandage has absorbent properties that help to keep the skin always dry, which is very convenient for tattoo-sensitive skins.

Its tubular shape allows the bandage to have different lengths and diameters to cover almost any part of the body.

For the first 24/48 hours after applying the tattoo, the skin may expel part of the ink (which will already be estimated by the tattooist) and such ink could adhere or attach the skin to the bandage. In such case, water should be applied to the bandage, which will easily detach from the skin.

In the present embodiment, the colour-fixing process may be done by steaming, whose main function is to fix the colours but also operates as a sanitizer, sterilizing the product, which is ideal for applying on the skin.

In a specific embodiment, the bandage would have the following configuration:
- 92% viscose over the total
- 5% elastane over the total, and
- 3% polyamide over the total

Once the tattooist has accomplished the artistic work on the skin, the tattooist will choose a bandage that is suitable to the size of the part of the body to be covered, such as a forearm.

Then, the tattooist will use a bandage with an inlet and an outlet, will introduce the bandage through the hand and place the bandage over the tattoo. If the skin is irritated, a dermatological cream may be applied on top of the bandage to relieve the irritation. One option is that said cream be already incorporated in the bandage by either microcapsules or by a coating.

This invention describes a novel protective bandage for tattoos and use thereof as a tattoo protector. The examples provided herein are not limitative of the present invention and shall therefore allow different applications and adaptations, all of which within the scope of the following claims.

## Claims

1. Tattoo protector bandage of the type having a tubular configuration, having at least one opening for introducing a member of the human body and forming an inner face which is in contact with the skin, and an outer face; **characterised in that** it comprises:
- 90-94% viscose, over the total, being the viscose obtained from cellulose found in wood paste;
- 4-6% elastane, over the total; and
- 2-4% polyamide, over the total.

2. Bandage according to claim 1, **characterised in that** it comprises a dermatological product in its inner face.

3. Bandage according to claim 1 or 2, for use as a tattoo protector.

## Patentansprüche

1. Tattoo-Schutzbandage in Schlauchform mit mindestens einer Öffnung zum Einführen eines menschlichen Körperteils, einer Innenseite, die mit der Haut in Berührung kommt und einer Außenseite, die wie folgt zusammengesetzt ist:
- 90-94% Viskose, bezogen auf die Gesamtmenge, wobei die Viskose aus der in der Holzpaste enthaltenen Zellulose gewonnen wird;
- 4-6% Elasthan, bezogen auf die Gesamtmenge; und
- 2-4% Polyamid, bezogen auf die Gesamtmenge.

2. Bandage nach Anspruch 1, **dadurch gekennzeichnet, dass** sie auf der Innenseite ein Hautpflegeprodukt enthält.

3. Bandage nach Anspruch 1 oder 2 zur Verwendung als Tattoo-Schutz.

## Revendications

1. Bandage protecteur de tatouage du type ayant une configuration tubulaire, ayant au moins une ouverture pour introduire un membre du corps humain et présentant une face interne qui est en contact avec la peau et une face externe, **caractérisé en ce qu'**il contient
- 90 à 94% de viscose au total, la viscose étant obtenue à partir de cellulose présente dans de la pâte à bois,
- 4 à 6% d'élastane au total et
- 2 à 4% de polyamide au total.

2. Bandage selon la revendication 1, **caractérisé en ce qu'**il contient un produit dermatologique dans sa face interne.

3. Bandage selon la revendication 1 ou 2, à utiliser comme protecteur de tatouage.
